# EUROPEAN PATENT SPECIFICATION

(11) **EP 4 213 622 B1**
(45) Date of publication and mention of the grant of the patent: **07.01.2026**
(21) Application number: 21794184.8
(22) Date of filing: 20.09.2021
(51) Int. Cl.: A01K 67/362

(54) **LARVAE SEPARATION SYSTEM AND METHOD OF SEPARATING LARVAE**
LARVENTRENNSYSTEM UND VERFAHREN ZUR LARVENTRENNUNG
SYSTÈME DE SÉPARATION DE LARVES ET PROCÉDÉ DE SÉPARATION DE LARVES

(30) Priority: 21.09.2020 FI 20205912
(43) Date of publication of application: 26.07.2023
(73) Proprietor: Volare Oy, 00101 Helsinki (FI)
(72) Inventor: TÄHTINEN, Matti, 00101 Helsinki (FI); PARVIAINEN, Tuure, 00101 Helsinki (FI)
(74) Representative: Laine IP Oy
(86) International application number: PCT/FI2021/050620
(87) International publication number: WO 2022/058660

(56) References cited:
- CN-U- 211 353 608
- US-A- 3 814 057

## Description

### FIELD

The present disclosure relates to apparatuses and methods for breeding of animals, particularly larvae (juvenile form of animal with indirect development, undergoing metamorphosis such as, insects or amphibians) for human, animal consumption or biodegradable material processing.

### BACKGROUND

Consumption and industrial exploitation of insect-based protein has gained popularity during the last decade. Apparatuses and methods for the production of such protein have been the subject of development not only on an industrial grade but on a household scale as well.

US 2017/0042131 A1, for example, discloses a system and method for breeding and harvesting insects for "Entomogaphy" purposes. The disclosed system and method provides a solution for separating larvae from other lifestages, and from frass, dirt, and carcasses as well as other detritus by employing sieving with different sized sieves. The apparatus comprises a light source for urging mating between adult insects at an early stage of breeding. The apparatus also comprises a motivation element in order to automate the separation process for collecting the larvae for consumption at a late stage of breeding.

The breeding of larvae is challenging primarily for the reason that the subject is a living creature. There is significant variance in the rate of development between different individuals. However, harvest should be performed only at a distinct stage of development, wherein it is advantageous that the variance in development is minimized at harvest. In an attempt to produce large volumes of larvae protein of uniform quality, the output of the larvae production should be as homogenous as possible in terms of development size.

US3814057A discloses an apparatus for separating negatively phototactic house fly (Musca domestica L.) larvae from chicken hen excreta and for providing a chamber for the subsequent pupation of the larvae. US3814057A discloses the features of the preamble of claim 1.

There remains a need to further develop apparatuses and methods for the production of larvae so as to output a homogenous batch of larvae.

### SUMMARY OF THE INVENTION

The invention is defined by the features of the independent claims. Some specific embodiments are defined in the dependent claims.

According to a first aspect of the present invention, there is provided a larvae separation system according to claim 1.

According to a second aspect of the present invention, there is provided according to claim 10 a method of separating living larvae in such a larvae separation system.

The disclosure comprises the following features:
- the second separation chamber comprises a second classifier screen with a plurality of openings of a second size;
- the second size is smaller than the first size;
- the second separation chamber is located underneath the first separation chamber;
- movement of larvae from the first separation chamber to the second separation chamber through the first classifier screen is assisted by gravity;
- the larvae separation system comprises a third separation chamber which;
- the third separation chamber is connected to the second separation chamber;
- the third separation chamber comprises a third volume which is separated from the second volume by the second classifier screen;
- the third separation chamber comprises a third classifier screen with a plurality of openings of a third size;
- the third size is smaller than the second size;
- the third separation chamber is located underneath the second separation chamber, wherein movement of larvae from the second separation chamber to the third separation chamber through the second classifier screen is assisted by gravity;
- the second separation chamber comprises a second light source configured to selectively illuminate the second volume for motivating the passage of larvae contained in the second separation chamber to a third separation chamber through the first classifier screen;
- the separation chambers each comprising an opaque frame laterally delimiting the respective volume with the respective classifier screen delimiting the bottom of the respective volume;
- the larvae separation system comprises a circulation chamber is connected to the second separation chamber or third separation chamber;
- the circulation chamber comprises a harvest volume which is separated from the second volume or the third volume by the second classifier screen or third classifier screen, respectively;
- the third separation chamber comprises a third light source configured to selectively illuminate the third volume for motivating the passage of larvae contained in the third separation chamber to the circulation chamber through the third classifier screen;
- the larvae separation system comprises a lid for delimiting the top of the first volume;
- the lid is opaque, and
- in which the a second screening is performed to the subset of said plurality of larvae contained in the second separation chamber by emitting light into the second volume, preferably onto the second classifier screen, for motivating a subset of the larvae contained in the second separation chamber to travel to the third separation chamber through the second classifier screen.

Considerable benefits are gained with aid of the present proposal. The system and method aim to decrease the standard deviation of the size and thus development stage of the larvae being bred. Precise management of larvae size helps to homogenize the output of the breeding process, whereby the larvae may be harvested in the optimal development stage, such as at the transition from larvae to pupae, so as to maximize the yield of protein. In other words, improved screening increases the cycle rate of the insect rearing process, whereby mortality rate may be reduced increasing yield. The proposed system and method contribute to the screening of larvae in the middle phase of breeding between mating and harvest so as to achieve a uniform and narrow distribution of development size.

### BRIEF DESCRIPTION OF THE DRAWINGS

In the following certain exemplary embodiments are described in greater detail with reference to the accompanying drawings, in which:
- FIGURE 1: illustrates a perspective view of a larvae separation system in accordance with at least some embodiments of the present invention;
- FIGURE 2: illustrates a sectional view of the larvae separation system of FIGURE 1, and
- FIGURE 3: illustrates a perspective view of the chambers of the larvae separation system of FIGURE 1 arranged adjacent to each other on a plane for illustrating the difference in classifier screen size.

### EMBODIMENTS

In the following certain embodiments will be described that make use of a general concept of separating young, e.g. less than or at most 7-day-old, larvae into similar development groups. The method is based on utilizing a combined mechanical separation and light source with, for example, a specific visible spectrum between 400 and 750 nm. Additionally or alternatively, additional light spectrum from UVA, B, C area between 100 and 400 nm can be applied. The proposed system and method is intended for use in tandem with and/or after a separate rearing process being performed in a separate or incorporated growth system (not shown in the FIGURES). The material produced may be used for human or animal consumption or as raw material for further processing, such as biodegradable material processing.

In the present context, especially arthropod larvae is foreseen as the subject, e.g. black soldier flies *(Hermetia illucens)* or houseflies (*Musca domestica*)*.*

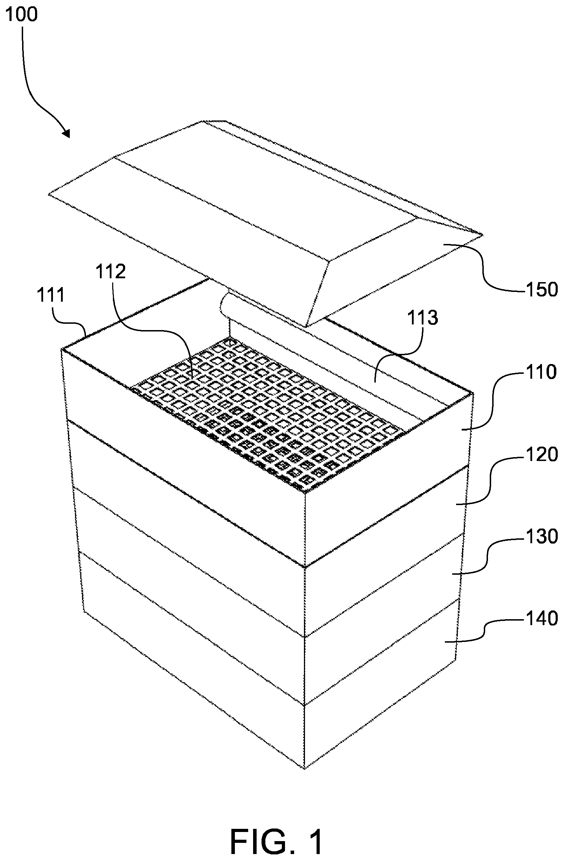

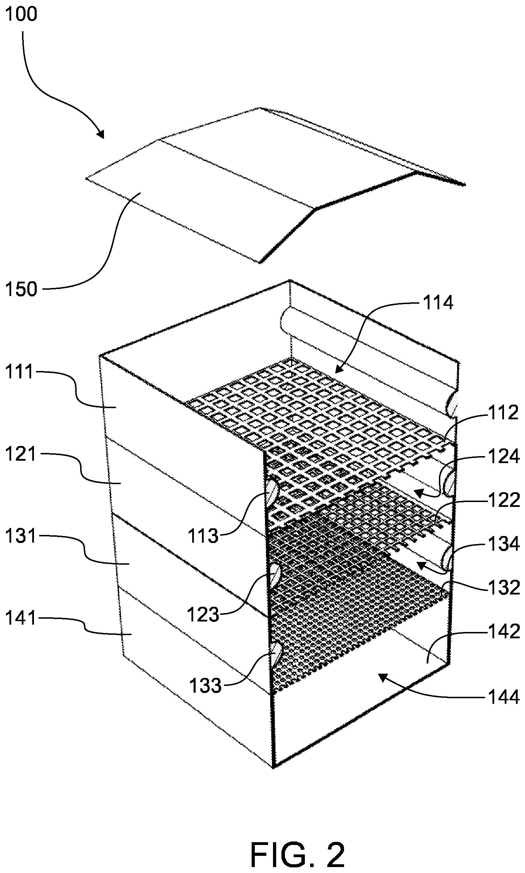

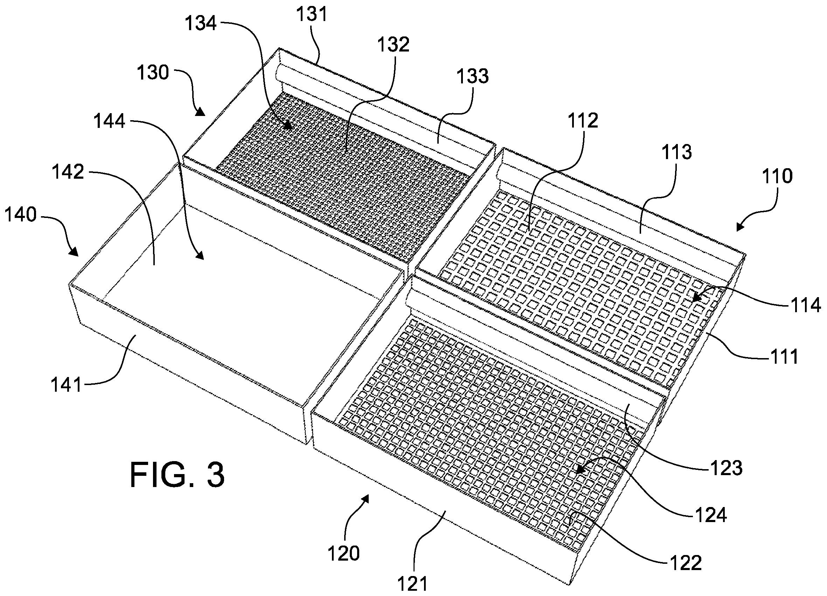

FIGURE 1 shows a larvae separation system 100 according to an exemplary embodiment designed to rear young larvae into harvestable larvae. The illustrated system 100 features a plurality of mutually superposed separation chambers 110, 120, 130, 140 and a lid 150 closing the system. A circulation chamber 140 is placed on the bottom with three separation chambers 130, 120, 110 placed on top in succession so as to classify living larvae between three different development stages. FIGURES 2 and 3 show the mechanical details of the chambers 110-140 in a more exposed manner. The separation system 100 is intended to receive a large plurality of larvae reared to a varying stage of development in a separate or interconnected rearing system (not shown in the FIGURES) and to separate the larvae into said stages of developments. The separation system 100 is intended to house the larvae for a relatively short period of time compared to the time spent in a growth system. A typical separation sequence will take less than an hour, for example 15 to 30 minutes.

The first separation chamber 110 is placed on top of the stack of chambers. The first chamber 110 is intended to receive a mixture of relatively young larvae and rearing substance. The first separation chamber 110 has a frame 111 which delimits the inner volume of the chamber 110, i.e. a first volume 114, laterally. The frame 111 is preferably opaque to prevent passage of light through the sides of the first separation chamber 110. The top of the first separation chamber 110 is exposed but may be closed with a lid 150 which is also preferably opaque for similar reasons. The inner volume 114 is delimited by a first classifier screen 112 on the bottom. In the present context the term classifier screen is used broadly to refer to not only mesh but all separators which include a plurality of openings of a certain size or a close size range. The classifier screen could, in other words, be constructed of a perforated plate with circular, oval, rectangular, or octagonal openings or openings with any other foreseeable shape. According to an exemplary embodiment, the first classifier screen 112 is a mesh with openings with dimensions in the range of 3 and 5 mm. Accordingly, only larvae smaller than the openings on the first classifier screen 112 may escape from the first separation chamber 110. The first classification by the first classifier screen 112 may only separate living larvae from dead ones and from other inanimate objects being contained in the rearing substance. The first classifier screen 112 may, for example, filter out foreign bodies from the larvae and rearing substance so as to spare the componentry used in a downstream processing stage.

The opening size of the first classifier screen 112 may be understood as the largest dimension of the openings in the first classifier screen 112.

The first separation chamber 110 is provided with a first light source 113 for selectively illuminating the inner volume of the chamber. According to the illustrated embodiment the light source 113 is an artificial light source, such an LED strip attached to the inner surface of the frame 111. Other alternative lighting arrangements are also foreseen, such as individual LEDs or light bulbs on the frame or an illuminator provided to the lid for a similar purpose. The light source may be configured to emit light in a visible light spectrum between 400 and 750 nm. Additionally, light spectrum from the UV area, such as between 100 and 400 nm, can be used. Alternatively, the light source could be a selectively opened opaque hatch covering an opening with a transparent window provided to the frame for selectively permitting passage of ambient light through the frame.

According to one embodiment the first light source 113 is set to illuminate the first chamber 111 with a light wavelength peaking at between 330 and 350 nm, particularly at 340 nm, or between 435 and 455 nm particularly at 445 nm, or between 495 and 515 nm, particularly at 505 nm. The light source 113 may exhibit intensity peaks at more than one of said wavelengths, for example at 340 ± 10 nm, 445 ± 10 nm, and/or 505 nm ± 10 nm.

The second separation chamber 120 is positioned below the first separation chamber 110 so as to assist passage of larvae between the separation chambers 110, 120 with gravity. The construction of the second chamber 120 is similar to the first separation chamber 110 with the exception that the classifier screen 122 of the second chamber 120 is tighter than the first classifier screen 112 of the first separation chamber 110. According to an exemplary embodiment, the second classifier screen 112 is a mesh with openings with dimensions in the range of 1 and 2 mm.

The opening size of the second classifier screen 122 may be understood as the largest dimension of the openings in the second classifier screen 122.

A second light source 123 of the second separation chamber 120 may emit light in an intensity or wavelength that is different to the first light source 113 of the first separation chamber 110. The light sources in the subsequent stages of separation may have a different intensity in that intensity may degrees with every stage of filtration so as to increase the contrast between darkness at the bottom of the system 100 and brightness at the top of the system 100 and to address the decreasing amount of rearing substance with each stage of separation. Alternatively or additionally the lighting may be constant or cyclic, i.e. targeted, to certain stages of separation. In constant lighting, the first stages are lit with more intensity than the latter stages. In cyclic lighting, the successive stages (separation chambers) are lit in turn. Each lighting cycle has a duration of a couple of minutes, e.g. five minutes.

According to one embodiment the second light source 123 is set to illuminate the second chamber 121 with a light wavelength peaking at between 330 and 350 nm, particularly at 340 nm, or between 435 and 455 nm particularly at 445 nm, or between 495 and 515 nm, particularly at 505 nm. The light source 123 may exhibit intensity peaks at more than one of said wavelengths, for example at 340 ± 10 nm, 445 ± 10 nm, and/or 505 nm ± 10 nm.

A third separation chamber 130 and any optional subsequent separation chamber (not shown in the FIGURES) is/are positioned below the second separation chamber 120. The construction of the third chamber 130 and any subsequent separation chamber (not shown in the FIGURES) is similar to the second separation chamber 120 with the exception that the classifier screen 132 of the third chamber 130 and any subsequent separation chamber (not shown in the FIGURES) is tighter than the classifier screen 122 of the second separation chamber 120. According to an exemplary embodiment, the third classifier screen 132 is a mesh with openings with dimensions in the range of 0.1 and 1 mm, particularly less than 1 mm. Also, the light source 133 of the third separation chamber 130 may emit light in an intensity or wavelength that is different to the light source 123 of the second separation chamber 120.

The opening size of the third classifier screen 132 may be understood as the largest dimension of the openings in the third classifier screen 132.

According to one embodiment the third light source 133 is set to illuminate the third chamber 131 with a light wavelength peaking at between 330 and 350 nm, particularly at 340 nm, or between 435 and 455 nm particularly at 445 nm, or between 495 and 515 nm, particularly at 505 nm. The light source 133 may exhibit intensity peaks at more than one of said wavelengths, for example at 340 ± 10 nm, 445 ± 10 nm, and/or 505 nm ± 10 nm.

The circulation chamber 140 on the bottom of the system 100 is, in the simplest form, a blind box with an open top. The circulation chamber 130 may include a drawer (not shown in the FIGURES) for extracting the output from the system 100. The chambers 110-140 may be attached to each other by a clamping mechanism (omitted from the FIGURES) or positively engaging fittings in the mechanical interface between subsequent frames 111-141 (omitted from the FIGURES).

The separation method with aid of such a larvae separation system 100 is straight-forward. First, larvae are placed on the classifier screen 112 of the first separation chamber 110 suspended in a rearing substance. With the lid 150 closed, the first volume 114 inside the first separation chamber 110 is dark. The separation process begins by illuminating the first volume by turning the light source 113 on. More specifically, the light is focused onto the first classifier screen 112. As larvae have a tendency of avoiding specific emitted wavelengths, the larvae which can fit through the openings of the first classifier screen 112 will crawl through to the second separation chamber 120 leaving larger and thus more mature larvae in the first separation chamber 110. By dropping into the second separation chamber 120 the relatively small larvae experience less light intensity, which decreases their motivation to proceed further in the system. The progression in the system 100 may be controlled by performing a cyclic illumination sequence, wherein only the first separation chamber 110 is illuminated for a period of time, e.g. five minutes, to complete the first stage of separation.

After the first stage of separation, the second light source 123 is illuminated for illuminating the second classifier screen 122 and the larvae growing thereon. If the separation is performed with cyclic illumination, the first light source 133 is turned off. Now the smaller larvae have motivation and ability to escape the second chamber 120 by crawling through the openings in the second classifier screen 122. Only the larvae that are too large to fit through the second classifier screen 122 are left in the second separation chamber 120.

The same is repeated in the optional third 130 and any subsequent separation chamber until the circulation chamber 140 contains very small larvae which may be recirculated back into the separation chamber 110, to a growing system, or discarded to provide evolutionary pressure to breed insects to grow faster, since slowest growing larvae can be removed.

With the classification performed, each chamber 110 has a plurality of larvae growing therein such classified to control for larvae size, shape and/or age. The effective classification will lead to a decrease in the standard deviation of the population age in the system which, in turn, leads to to more uniform completion of the rearing cycle thus increasing the yield of production. The classified development groups are eventually harvested for post-processing and/or consumption.

The passage of larvae between chambers 110-140 may be advanced with additional aids. The system 100 may, for example, be placed on a horizontally and/or vertically vibrating platform or with water or air flow (not shown in the FIGURES).

The system herein described may be varied according to the screening needs. The third separation stage, i.e. the third separation chamber, may be omitted, if the two stages are sufficient. Alternatively, more stages, i.e. separation chambers may be added between the third separation chamber and the circulation chamber. Additionally or alternatively the circulation chamber may be omitted, wherein the smallest larvae may escape directly to a discard bin or to a growth chamber. Additionally or alternatively the opening sizes herein described may be adjusted for tailoring the screening to the desired development stage.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

### REFERENCE SIGNS LIST

| No. | Feature | No. | Feature |
|---|---|---|---|
| 100 | larvae separation system | 130 | third separation chamber |
| 110 | first separation chamber | 131 | frame |
| 111 | frame | 132 | classifier screen |
| 112 | classifier screen | 133 | third (artificial) light source |
| 113 | first (artificial) light source | 134 | third volume |
| 114 | first volume | 140 | circulation chamber |
| 120 | second separation chamber | 141 | frame |
| 121 | frame | 142 | bottom |
| 122 | classifier screen | 144 | harvest volume |
| 123 | second (artificial) light source | 150 | lid |
| 124 | second volume | | |

### CITATION LIST

US 2017/0042131 A1

## Claims

1. A larvae separation system (100) comprising:
- a first separation chamber (110) comprising:
∘ a first classifier screen (112) with a plurality of openings of a first size, which is between 3 and 5 mm, and
∘ a first volume (114), which is at least partly delimited by the first classifier screen (112),
- a second separation chamber (120) connected to the first chamber (110) and comprising a second volume (124), wherein the second separation chamber (120) comprising a second classifier screen (122) with a plurality of openings of a second size, which is between 1 and 2 mm, and wherein the first volume (114) and the second volume (124) are separated by the first classifier screen (112), and
- a first light source (113),
**characterized in that**:
- the first light source (113) is configured to selectively illuminate the first volume (114) for motivating the passage of larvae contained in the first separation chamber (110) to the second separation chamber (120) through the first classifier screen (112)
- the larvae separation system (100) comprises a third separation chamber (130), which is connected to the second separation chamber (120) and which comprises:
∘ a third volume (130), which is separated from the second volume (120) by the second classifier screen (122), and
∘ a third classifier screen (132) with a plurality of openings of a third size, which is between 0.1 and 1 mm, and **in that**
- the second separation chamber (120) comprises a second light source (123) configured to selectively illuminate the second volume (124) for motivating the passage of larvae contained in the second separation chamber (120) to the third separation chamber (130) through the second classifier screen (122).

2. The larvae separation system (100) according to claim 1, wherein the second separation chamber (120) is located underneath the first separation chamber (110), wherein movement of larvae from the first separation chamber (110) to the second separation chamber (120) through the first classifier screen (112) is assisted by gravity.

3. The larvae separation system (100) according to claims 1 or 2, wherein the third separation chamber (130) is located underneath the second separation chamber (120), wherein movement of larvae from the second separation chamber (120) to the third separation chamber (130) through the second classifier screen (122) is assisted by gravity.

4. The larvae separation system (100) according to any one of the preceding claims, wherein the separation chambers (110, 120, 130) each comprising an opaque frame (111, 121, 131) laterally delimiting the respective volume (114, 124, 134) with the respective classifier screen (112, 122, 132) delimiting the bottom of the respective volume (114, 124, 134).

5. The larvae separation system (100) according to any one of the preceding claims, wherein the larvae separation system (100) comprises a circulation chamber (140) which:
- is connected to the third separation chamber (130) and which
- comprises a harvest volume (144) which is separated from the third volume (134) by the third classifier screen (132).

6. The larvae separation system (100) according to claims 5, wherein the third separation chamber (130) comprises a third light source (133) configured to selectively illuminate the third volume (134) for motivating the passage of larvae contained in the third separation chamber (130) to the circulation chamber (140) through the third classifier screen (132).

7. The larvae separation system (100) according to any one of the preceding claims, wherein the light sources (113, 123, 133) are configured to illuminate the respective volumes (114, 124, 134) independently from each other.

8. The larvae separation system (100) according to any one of the preceding claims, wherein each one or one or more of the light sources (113, 123, 133) is/are configured to emit light wavelengths peaking at 340 ± 10 nm, 445 ± 10 nm, and/or 505 nm ± 10 nm.

9. The larvae separation system (100) according to any one of the preceding claims, wherein the larvae separation system (100) comprises a lid (150) for delimiting the top of the first volume (114), wherein the lid (150) is preferably opaque.

10. A method of separating living larvae in a larvae separation system (100) in accordance with any one of the preceding claims, the method comprising:
- providing a plurality of larvae and rearing substance into the first separation chamber (110);
- performing a first screening to the plurality of larvae by emitting light into the first volume (114), preferably onto the first classifier screen (112), for motivating a subset of said plurality of larvae contained in the first chamber (110) to travel to the second separation chamber (120) through the first classifier screen (112),
- performing a second screening to the subset of said plurality of larvae contained in the second separation chamber (120) by emitting light into the second volume (124), preferably onto the second classifier screen (122), for motivating a subset of the larvae contained in the second separation chamber (120) to travel to the third separation chamber (130) through the second classifier screen (122), and
- harvesting the screened pluralities of larvae of different development groups.

## Patentansprüche

1. Larventrennsystem (100) umfassend:
- eine erste Trennkammer (110) umfassend:
- ein erstes Klassiersieb (112) mit einer Vielzahl von Öffnungen einer ersten Größe, die zwischen 3 und 5 mm liegt, und
- ein erstes Volumen (114), das zumindest teilweise durch das erste Klassiersieb (112) eingegrenzt wird,
- eine zweite Trennkammer (120), die mit der ersten Kammer (110) verbunden ist und ein zweites Volumen (124) umfasst, wobei die zweite Trennkammer (120) ein zweites Klassiersieb (122) mit einer Vielzahl von Öffnungen einer zweiten Größe zwischen 1 und 2 mm umfasst und wobei das erste Volumen (114) und das zweite Volumen (124) durch das erste Klassiersieb (112) getrennt sind, und
- eine erste Lichtquelle (113),
**dadurch gekennzeichnet, dass**:
- die erste Lichtquelle (113) so konfiguriert ist, dass sie selektiv das erste Volumen (114) beleuchtet, um den Durchgang von in der ersten Trennkammer (110) enthaltenen Larven durch das erste Klassiersieb (112) in die zweite Trennkammer (120) zu veranlassen,
- das Larventrennsystem (100) eine dritte Trennkammer (130) umfasst, die mit der zweiten Trennkammer (120) verbunden ist und Folgendes umfasst:
- ein drittes Volumen (130), das durch das zweite Klassiersieb (122) vom zweiten Volumen (120) getrennt ist, und
- ein drittes Klassiersieb (132) mit einer Vielzahl von Öffnungen einer dritten Größe, die zwischen 0,1 und 1 mm liegt, und dass
- die zweite Trennkammer (120) eine zweite Lichtquelle (123) umfasst, die so konfiguriert ist, dass sie selektiv das zweite Volumen (124) beleuchtet, um den Durchgang von in der zweiten Trennkammer (120) enthaltenen Larven durch das zweite Klassiersieb (122) in die dritte Trennkammer (130) zu veranlassen.

2. Larventrennsystem (100) nach Anspruch 1, wobei die zweite Trennkammer (120) unterhalb der ersten Trennkammer (110) angeordnet ist, wobei die Bewegung von Larven von der ersten Trennkammer (110) zur zweiten Trennkammer (120) durch das erste Klassiersieb (112) durch Schwerkraft unterstützt wird.

3. Larventrennsystem (100) nach Anspruch 1 oder 2, wobei die dritte Trennkammer (130) unterhalb der zweiten Trennkammer (120) angeordnet ist, wobei die Bewegung von Larven von der zweiten Trennkammer (120) zur dritten Trennkammer (130) durch das zweite Klassiersieb (122) durch Schwerkraft unterstützt wird.

4. Larventrennsystem (100) nach einem der vorstehenden Ansprüche, wobei die Trennkammern (110, 120, 130) jeweils einen undurchsichtigen Rahmen (111, 121, 131) umfassen, der das jeweilige Volumen (114, 124, 134) seitlich eingrenzt, wobei das jeweilige Klassiersieb (112, 122, 132) den Boden des jeweiligen Volumens (114, 124, 134) abgrenzt.

5. Larventrennsystem (100) nach einem der vorstehenden Ansprüche, wobei das Larventrennsystem (100) eine Zirkulationskammer (140) umfasst, die:
- mit der dritten Trennkammer (130) verbunden ist und die
- ein Erntevolumen (144) umfasst, das durch das dritte Klassiersieb (132) vom dritten Volumen (134) getrennt ist.

6. Larventrennsystem (100) nach Anspruch 5, wobei die dritte Trennkammer (130) eine dritte Lichtquelle (133) umfasst, die so konfiguriert ist, dass sie selektiv das dritte Volumen (134) beleuchtet, um den Durchgang von in der dritten Trennkammer (130) enthaltenen Larven durch das dritte Klassiersieb (132) in die Zirkulationskammer (140) zu veranlassen.

7. Larventrennsystem (100) nach einem der vorstehenden Ansprüche, wobei die Lichtquellen (113, 123, 133) so konfiguriert sind, dass sie die jeweiligen Volumina (114, 124, 134) unabhängig voneinander beleuchten.

8. Larventrennsystem (100) nach einem der vorstehenden Ansprüche, wobei jede der oder eine oder mehrere der Lichtquellen (113, 123, 133) so konfiguriert sind, dass sie Lichtwellenlängen mit Spitzenwerten bei 340 ±10 nm, 445 ±10 nm und/oder 505 nm ±10 nm aussenden.

9. Larventrennsystem (100) nach einem der vorstehenden Ansprüche, wobei das Larventrennsystem (100) einen Deckel (150) zum Abgrenzen des oberen Teils des ersten Volumens (114) umfasst, wobei der Deckel (150) bevorzugt undurchsichtig ist.

10. Verfahren zum Abtrennen lebender Larven in einem Larventrennsystem (100) nach einem der vorstehenden Ansprüche, wobei das Verfahren Folgendes umfasst:
- Bereitstellen einer Vielzahl von Larven und Aufzuchtmaterial in der ersten Trennkammer (110);
- Durchführen einer ersten Siebung der Vielzahl von Larven durch Aussenden von Licht in das erste Volumen (114), bevorzugt auf das erste Klassiersieb (112), um eine Teilmenge der in der ersten Kammer (110) enthaltenen Vielzahl von Larven zu veranlassen, durch das erste Klassiersieb (112) in die zweite Trennkammer (120) zu gelangen,
- Durchführen einer zweiten Siebung der in der zweiten Trennkammer (120) enthaltenen Vielzahl von Larven durch Aussenden von Licht in das zweite Volumen (124), bevorzugt auf das zweite Klassiersieb (122), um eine Teilmenge der in der zweiten Trennkammer (120) enthaltenen Larven zu motivieren, durch das zweite Klassiersieb (122) in die dritte Trennkammer (130) zu gelangen, und
- Ernten der gesiebten Vielzahlen von Larven verschiedener Entwicklungsstadien.

## Revendications

1. Système de séparation de larves (100) comprenant :
- une première chambre de séparation (110) comprenant :
- un premier tamis classificateur (112) avec une pluralité d'ouvertures d'une première taille, qui est comprise entre 3 et 5 mm, et
- un premier volume (114), qui est au moins partiellement délimité par le premier tamis classificateur (112),
- une deuxième chambre de séparation (120) reliée à la première chambre (110) et comprenant un deuxième volume (124), dans lequel la deuxième chambre de séparation (120) comprend un deuxième tamis classificateur (122) avec une pluralité d'ouvertures d'une deuxième taille, qui est comprise entre 1 et 2 mm, et dans lequel le premier volume (114) et le deuxième volume (124) sont séparés par le premier tamis classificateur (112), et
- une première source de lumière (113),
**caractérisé en ce que** :
- la première source de lumière (113) est configurée pour éclairer sélectivement le premier volume (114) afin d'inciter le passage des larves contenues dans la première chambre de séparation (110) vers la deuxième chambre de séparation (120) à travers le premier tamis classificateur (112),
- le système de séparation de larves (100) comprend une troisième chambre de séparation (130), qui est reliée à la deuxième chambre de séparation (120) et qui comprend :
- un troisième volume (130), qui est séparé du deuxième volume (120) par le deuxième tamis classificateur (122), et
- un troisième tamis classificateur (132) avec une pluralité d'ouvertures d'une troisième taille, qui est comprise entre 0,1 et 1 mm, et **en ce que**
- la deuxième chambre de séparation (120) comprend une deuxième source de lumière (123) configurée pour éclairer sélectivement le deuxième volume (124) afin d'inciter le passage des larves contenues dans la deuxième chambre de séparation (120) vers la troisième chambre de séparation (130) à travers le deuxième tamis classificateur (122).

2. Système de séparation de larves (100) selon la revendication 1, dans lequel la deuxième chambre de séparation (120) est située sous la première chambre de séparation (110), dans lequel le mouvement des larves de la première chambre de séparation (110) à la deuxième chambre de séparation (120) à travers le premier tamis classificateur (112) est assisté par la gravité.

3. Système de séparation de larves (100) selon les revendications 1 ou 2, dans lequel la troisième chambre de séparation (130) est située sous la deuxième chambre de séparation (120), dans lequel le mouvement des larves de la deuxième chambre de séparation (120) à la troisième chambre de séparation (130) à travers le deuxième tamis classificateur (122) est assisté par la gravité.

4. Système de séparation de larves (100) selon l'une quelconque des revendications précédentes, dans lequel les chambres de séparation (110, 120, 130) comprennent chacune un cadre opaque (111, 121, 131) délimitant latéralement le volume (114, 124, 134) respectif avec le tamis classificateur (112, 122, 132) respectif délimitant le fond du volume (114, 124, 134) respectif.

5. Système de séparation de larves (100) selon l'une quelconque des revendications précédentes, dans lequel le système de séparation de larves (100) comprend une chambre de circulation (140) qui :
- est relié à la troisième chambre de séparation (130) et qui
- comprend un volume de récolte (144) qui est séparé du troisième volume (134) par le troisième tamis classificateur (132).

6. Système de séparation de larves (100) selon la revendication 5, dans lequel la troisième chambre de séparation (130) comprend une troisième source de lumière (133) configurée pour éclairer sélectivement le troisième volume (134) afin d'inciter le passage des larves contenues dans la troisième chambre de séparation (130) vers la chambre de circulation (140) à travers le troisième tamis classificateur (132).

7. Système de séparation de larves (100) selon l'une quelconque des revendications précédentes, dans lequel les sources de lumière (113, 123, 133) sont configurées pour éclairer les volumes (114, 124, 134) respectifs indépendamment les uns des autres.

8. Système de séparation de larves (100) selon l'une quelconque des revendications précédentes, dans lequel chacune ou une ou plusieurs des sources de lumière (113, 123, 133) est/sont configurée(s) pour émettre des longueurs d'onde de lumière atteignant un maximum de 340 ± 10 nm, 445 ± 10 nm et/ou 505 nm ± 10 nm.

9. Système de séparation de larves (100) selon l'une quelconque des revendications précédentes, dans lequel le système de séparation de larves (100) comprend un couvercle (150) pour délimiter le haut du premier volume (114), dans lequel le couvercle (150) est de préférence opaque.

10. Procédé de séparation de larves vivantes dans un système de séparation de larves (100) selon l'une quelconque des revendications précédentes, le procédé comprenant :
- la fourniture d'une pluralité de larves et de substance d'élevage dans la première chambre de séparation (110) ;
- la réalisation d'un premier criblage sur la pluralité de larves en émettant de la lumière dans le premier volume (114), de préférence sur le premier tamis classificateur (112), afin d'inciter un sous-ensemble de ladite pluralité de larves contenues dans la première chambre (110) à se déplacer vers la deuxième chambre de séparation (120) à travers le premier tamis classificateur (112),
- la réalisation d'un second criblage sur le sous-ensemble de ladite pluralité de larves contenues dans la deuxième chambre de séparation (120) en émettant de la lumière dans le deuxième volume (124), de préférence sur le deuxième tamis classificateur (122), afin d'inciter un sous-ensemble des larves contenues dans la deuxième chambre de séparation (120) à se déplacer vers la troisième chambre de séparation (130) à travers le deuxième tamis classificateur (122), et
- la récolte des pluralités de larves criblées de différents groupes de développement.
